# EUROPEAN PATENT APPLICATION

(11) **EP 2 345 651 A1**
(43) Date of publication of application: **20.07.2011**
(21) Application number: 09819110.9
(22) Date of filing: 29.09.2009
(51) Int. Cl.: C07D 409/14, C01B 31/02, H01L 51/42

(54) **FULLERENE DERIVATIVE, COMPOSITION, AND ORGANIC PHOTOELECTRIC CONVERSION ELEMENT**

(30) Priority: 06.10.2008 JP 2008259471; 10.02.2009 JP 2009028128; 21.05.2009 JP 2009122910
(71) Applicant: Sumitomo Chemical Company, Limited, Tokyo 104-8260 (JP); National University Corporation Tottori University, Tottori-shi Tottori 680-8550 (JP)
(72) Inventor: ITOH Toshiyuki, Tottori-shi Tottori 680-8550 (JP); UETANI Yasunori, Tsukuba-shi Ibaraki 305-0045 (JP)
(74) Representative: Vossius & Partner
(86) International application number: PCT/JP2009/066978
(87) International publication number: WO 2010/041575

(57) **Abstract**

The present invention provides a fullerene derivative represented by the following formula (1). 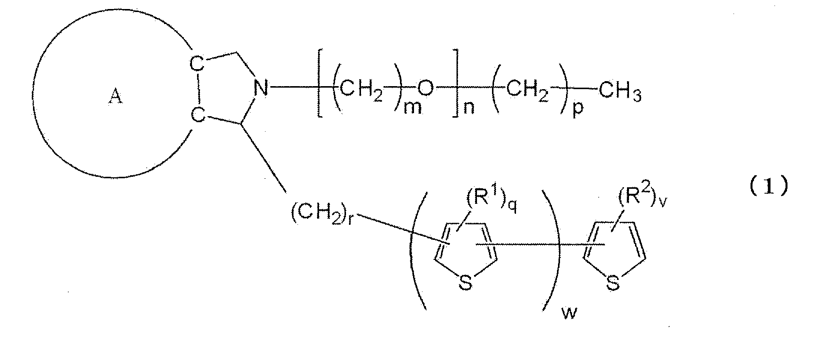 [In formula (1), a ring A represents a fullerene skeleton having 60 or more carbon atoms; R¹ and R² are the same as or different from each other, and each represent a halogen atom, an alkyl group, an alkoxy group or an aryl group (wherein the whole or a part of hydrogen atoms belonging to the alkyl group, the alkoxy group or the aryl group may be substituted with halogen atoms or a halogen atom); m represents an integer of 1 to 6, n represents an integer of 1 to 4, p represents an integer of 0 to 5, w represents an integer of 1 to 6, r represents an integer of 0 to 4, q represents an integer of 0 to 2 and v represents an integer of 0 to 3; when there are a plurality of R¹s, the R¹s may be the same as or different from each other; when there are a plurality of R²s the R²s may be the same as or different from each other; when there are a plurality of m's, the m's may be the same as or different from each other; and when there are a plurality of q's, the q's may be the same as or different from each other.]

## Description

### Technical Field

The present invention relates to a fullerene derivative, a composition and an organic photoelectric conversion element using these.

### Background Art

Organic semiconductor materials having charge (electron and hole) transportability have been investigated for application to organic photoelectric conversion elements (such as organic solar cells and optical sensors); for example, organic solar cells using fullerene derivatives have been investigated. As such a fullerene derivative, for example, [6,6]-phenyl C61-butyric acid methyl ester (hereinafter, sometimes referred to as "[60]-PCBM") has been known (see Non **Patent Literature 1).**

### Citation List

### Non Patent Literature

[Non Patent Literature 1] Advanced Functional Materials, Vol. 13 (2003) p. 85.

### Summary of Invention

### Problems to be Solved by the Invention

However, there is a problem that an organic photoelectric conversion element comprising [60]-PCBM does not necessarily have a sufficient conversion efficiency.

Accordingly, the present invention takes as its object the provision of a fullerene derivative capable of producing an organic photoelectric conversion element having a high conversion efficiency, a composition comprising the fullerene derivative and an organic photoelectric conversion element using these.

### Means for Solving the Problems

The present invention provides a fullerene derivative represented by the following formula (1). 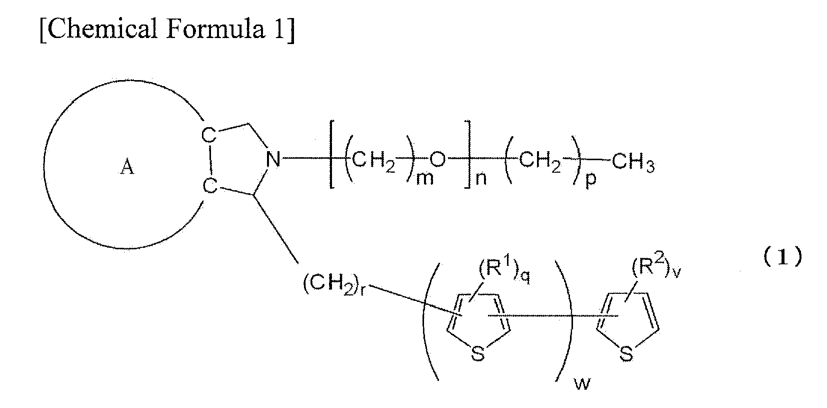 [In formula (1), a ring A represents a fullerene skeleton having 60 or more carbon atoms; R¹ and R² are the same as or different from each other, and each represent a halogen atom, an alkyl group, an alkoxy group or an aryl group (wherein the whole or a part of hydrogen atoms belonging to the alkyl group, the alkoxy group or the aryl group may be substituted with halogen atoms or a halogen atom); m represents an integer of 1 to 6, n represents an integer of 1 to 4, p represents an integer of 0 to 5, w represents an integer of 1 to 6, r represents an integer of 0 to 4, q represents an integer of 0 to 2 and v represents an integer of 0 to 3; when there are a plurality of R¹s, the R¹s may be the same as or different from each other; when there are a plurality of R²s, the R²s may be the same as or different from each other; when there are a plurality of m's, the m's may be the same as or different from each other; and when there are a plurality of q's, the q's may be the same as or different from each other.]

The present invention provides a composition comprising the above-described fullerene derivative and an electron-donating compound. In the composition of the present invention, the electron-donating compound is preferably a polymer compound.

The present invention also provides an organic photoelectric conversion element having a pair of electrodes, at least one of the electrodes being transparent or translucent, and an organic layer disposed between the pair of electrodes, wherein the organic layer comprises the fullerene derivative of the present invention. The present invention further provides an organic photoelectric conversion element having a pair of electrodes, at least one of the electrodes being transparent or translucent, and an organic layer disposed between the pair of electrodes, wherein the organic layer comprises the composition of the present invention.

### Effect of the Invention

According to the present invention, it is possible to provide a fullerene derivative capable of producing an organic photoelectric conversion element having a high conversion efficiency, a composition comprising the fullerene derivative and an organic photoelectric conversion element using these. The organic photoelectric conversion element having a layer comprising the fullerene derivative of the present invention is high in conversion efficiency and hence suitable for use in an organic thin film solar cell or for use in an organic optical sensor, and the present invention is extremely useful industrially.

### Embodiments for Carrying Out the Invention

Hereinafter, the present invention is described in detail.

### <Fullerene Derivative>

The fullerene derivative of the present invention is represented by the above formula (1).

In formula (1), the ring A represents a fullerene skeleton having 60 or more carbon atoms. The number of the carbon atoms in the fullerene skeleton (carbon cluster) is preferably 960 or less, more preferably 240 or less and furthermore preferably 96 or less. Examples of the fullerene skeleton having 60 to 96 carbon atoms include the C₆₀, C₇₀, C₇₆, C₇₈, C₈₀, C₈₂, C₈₄, C₈₆, C₈₈, C₉₀, C₉₂, C₉₄ or C₉₆ fullerene skeleton. From the viewpoint of the raw material availability, the ring A is preferably C₆₀ fullerene or C₇₀ fullerene.

In formula (1), R¹ and R² each independently represent a halogen atom, an alkyl group, an alkoxy group or an aryl group. The hydrogen atom or hydrogen atoms contained in the alkyl group, the alkoxy group or the aryl group may be substituted with a halogen atom or halogen atoms. When there are a plurality of R¹s, the R¹s may be the same as or different from each other. When there are a plurality of R²s, the R²s may be the same as or different from each other.

In formula (1), the alkyl group represented by R¹ or R² usually has 1 to 20 carbon atoms, and may be linear or branched, or a cycloalkyl group. Specific examples of the alkyl group include a methyl group, an ethyl group, a n-propyl group, an i-propyl group, a n-butyl group, an i-butyl group, a t-butyl group, a s-butyl group, a 3-methylbutyl group, a n-pentyl group, a n-hexyl group, a 2-ethylhexyl group, a n-heptyl group, a n-octyl group, a n-nonyl group, a n-decyl group and a n-lauryl group. The hydrogen atom or hydrogen atoms in the alkyl groups may be substituted with a halogen atom or halogen atoms, and specific examples of such halogen-substituted alkyl groups include a monohalomethyl group, a dihalomethyl group, a trihalomethyl group and a pentahaloethyl group. The hydrogen atom or hydrogen atoms is/are substituted preferably with a fluorine atom or fluorine atoms among a halogen atom or halogen atoms. Examples of the alkyl groups in which a hydrogen atom or hydrogen atoms is/are substituted with a fluorine atom or fluorine atoms include a trifluoromethyl group, a pentafluoroethyl group, a perfluorobutyl group, a perfluorohexyl group and a perfluorooctyl group.

In formula (1), the alkoxy group represented by R¹ or R² usually has 1 to 20 carbon atoms, may be linear or branched, or a cycloalkyloxy group. Specific examples of the alkoxy group include a methoxy group, an ethoxy group, a n-propyloxy group, an i-propyloxy group, a n-butoxy group, an i-butoxy group, a s-butoxy group, a t-butoxy group, a n-pentyloxy group, a n-hexyloxy group, a cyclohexyloxy group, a n-heptyloxy group, a n-octyloxy group, a 2-ethylhexyloxy group, a n-nonyloxy group, a n-decyloxy group, a 3,7-dimethyloctyloxy group and a n-lauryloxy group. The hydrogen atom or hydrogen atoms in the alkoxy groups may be substituted with a halogen atom or halogen atoms. The hydrogen atom or hydrogen atoms is/are substituted preferably with a fluorine atom or fluorine atoms among a halogen atom or halogen atoms. Examples of the alkoxy groups in which the hydrogen atom or hydrogen atoms is/are substituted with a fluorine atom or fluorine atoms include a trifluoromethoxy group, a pentafluoroethyoxy group, a perfluorobutoxy group, a perfluorohexyl group and a perfluorooctyl group.

In formula (1), the aryl group represented by R¹ or R² usually has 6 to 60 carbon atoms, and may have a substituent or substituents. Examples of the substituent belonging to the aryl group include a linear or branched alkyl group having 1 to 20 carbon atoms or a cycloalkyl group having 1 to 20 carbon atoms, or an alkoxy group containing, in the structure thereof, a linear or branched alkyl group having 1 to 20 carbon atoms or a cycloalkyl group having 1 to 20 carbon atoms. Specific examples of the aryl group include a phenyl group, a C₁ to C₂ alkoxyphenyl group (C₁ to C₁₂ means that the number of carbon atoms is 1 to 12; hereinafter, this is also the case), a C₁ to C₁₂ alkylphenyl group, a 1-naphthyl group and a 2-naphthyl group. As the aryl group, an aryl group having 6 to 20 carbon atoms is preferable, and a C₁ to C₁₂ alkoxyphenyl group and a C₁ to C₁₂ alkylphenyl group are more preferable. The hydrogen atom or hydrogen atoms in the aryl group may be substituted with a halogen atom or halogen atoms. The hydrogen atom or hydrogen atoms is/are substituted preferably with a fluorine atom or fluorine atoms among a halogen atom or halogen atoms.

In formula (1), examples of the halogen atom represented by R¹ or R² include a fluorine atom, a chlorine atom, a bromine atom and an iodine atom. From the viewpoint of the conversion efficiency, a fluorine atom is preferable.

In formula (1), m represents an integer of 1 to 6, and is preferably 2 from the viewpoint of the easiness in obtaining the raw material. When there a plurality of m's, the m's may be the same as or different from each other. And, p represents an integer of 0 to 5, and is preferably an integer of 0 to 3 from the viewpoint of the charge transportability. And, n represents an integer of 1 to 4, r represents an integer of 0 to 4, w represents an integer of 1 to 6, q represents an integer of 0 to 2 and v represents an integer of 0 to 3.

In the fullerene derivative represented by formula (1), specific examples of the C60 fullerene derivative include the compounds respectively represented by the following formulas (la) to (1g).

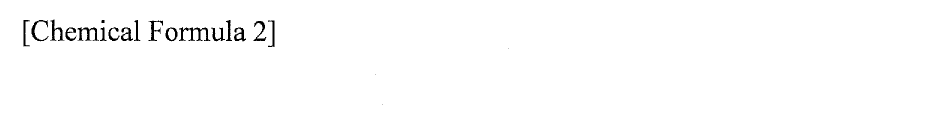

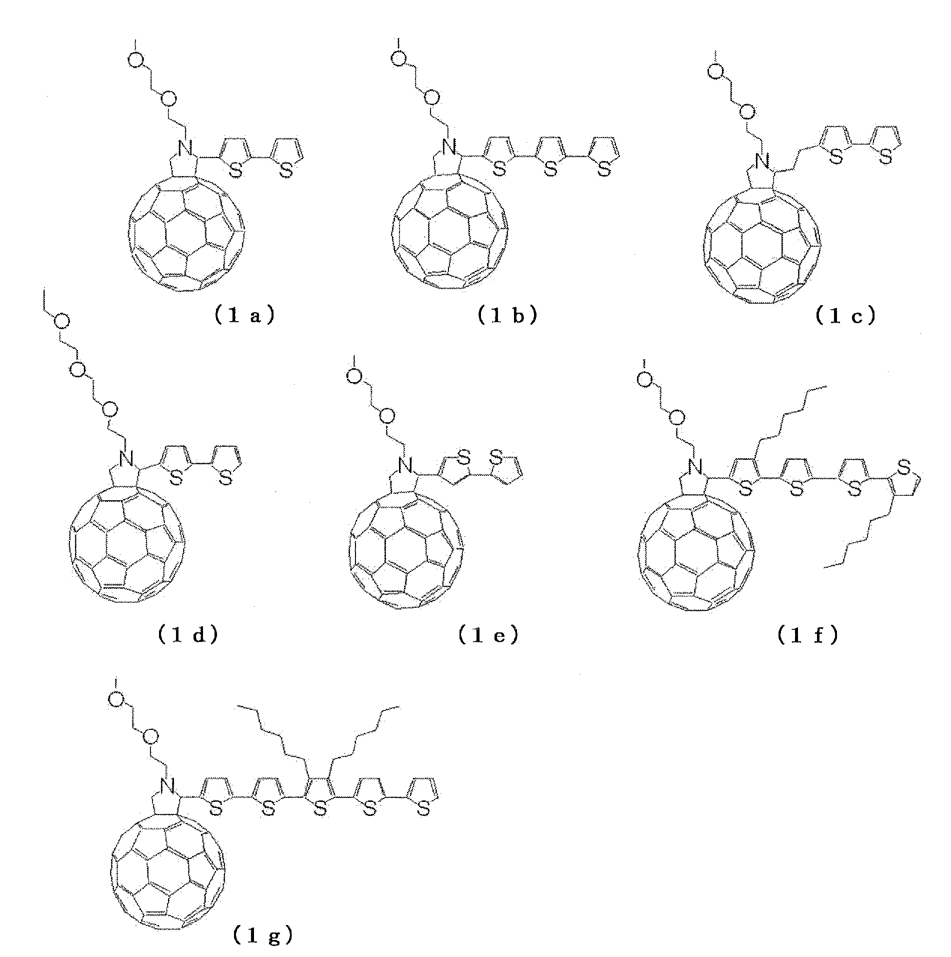

In the fullerene derivative represented by formula (1), specific examples of the C70 fullerene derivative include the compound represented by the following formula (1h). 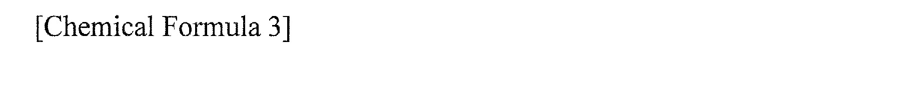 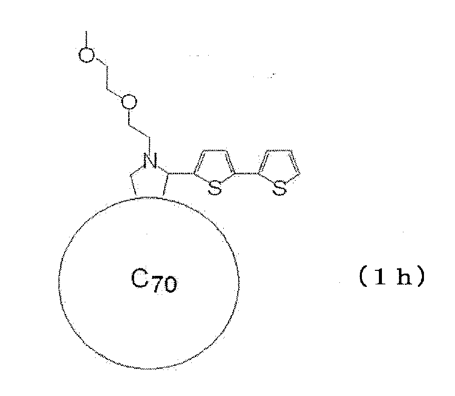 In formula (1h), the C70 ring represents a fullerene ring having 70 carbon atoms.

Of the fullerene derivatives represented by formula (1), from the viewpoint of the conversion efficiency, the fullerene derivative represented by the following formula (2) is preferable. 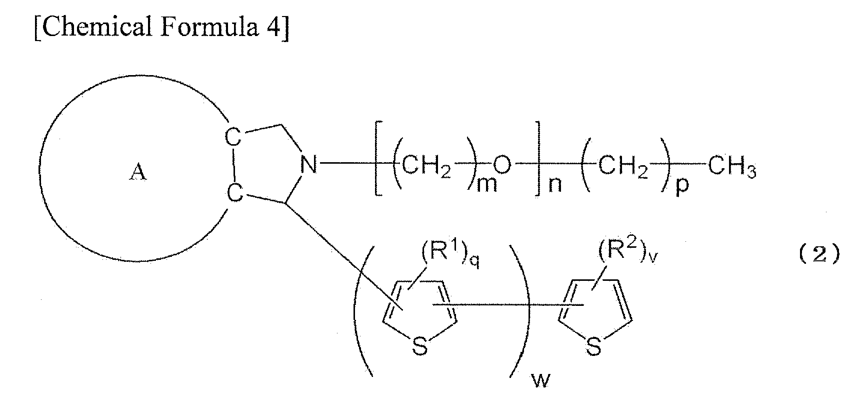 In formula (2), R¹, R², m, n, p, q, v and w represent the same meanings as described above.

In the above formula (2), from the viewpoint of the conversion efficiency, m and n are each preferably 2, p is preferably 0, q and v are each independently preferably 0 to 2, and w is preferably 1 to 4. R¹ and R² are each independently preferably an alkyl group having 1 to 12 carbon atoms. The hydrogen atom or hydrogen atoms contained in the alkyl group may be substituted with a fluorine atom or fluorine atoms.

In formula (2), the ring A represents a fullerene skeleton having 60 or more carbon atoms, and from the viewpoint of the raw material availability, C₆₀ fullerene or C₇₀ fullerene is preferable.

As the synthesis method of the fullerene derivative represented by formula (1), it is possible to use, for example, a 1,3-dipolar cycloaddition reaction (the Prato reaction, Accounts of Chemical Research, Vol. 31 (1998), pp. 519 to 526) between an iminium cation produced by the decarboxylation from an imine produced from a glycine derivative and an aldehyde compound, and fullerene.

Examples of the glycine derivative used herein include N-methoxymethylglycine and N-(2-(2-methoxyethoxy)ethyl)glycine. The used amount of the glycine derivative falls usually in a range from 0.1 to 10 mol and preferably in a range from 0.5 to 3 mol in relation to 1 mol of fullerene.

Examples of the aldehyde compound, which is another raw material for the substituent, include 2-thienyl-2-thiophenecarbaldehyde. The used amount of the aldehyde compound is usually in a range from 0.1 to 10 mol and preferably in a range from 0.5 to 4 mol in relation to 1 mol of fullerene.

Usually this reaction is performed in a solvent. As the solvent, there are used solvents inert to the reaction, such as toluene, xylene, hexane, octane and chlorobenzene. The used amount of the solvent falls usually in a range from 1 to 100000 parts by weight in relation to 1 part by weight of fullerene.

In performing the reaction, for example, a glycine derivative, an aldehyde compound and fullerene have only to be mixed together in a solvent, heated and allowed to react with each other, and the reaction temperature is usually in a range from 50 to 350°C. The reaction time is usually from 30 minutes to 50 hours. On completion of the reaction under heating, the reaction mixture is allowed to cool down to room temperature, then the solvent is distilled off under reduced pressure with a rotary evaporator, the obtained solid product is separated and purified by silica gel flush column chromatography, and thus the intended fullerene derivative can be obtained.

### <Composition>

The composition of the present invention comprises the fullerene derivative and the electron-donating compound.

From the view point of the coatability, the electron-donating compound is preferably a polymer compound. Examples of the polymer compound include: polyvinylcarbazole and derivatives thereof; polysilane and derivatives thereof, polysiloxane derivatives each having an aromatic amine in the side chain or the main chain; polyaniline and derivatives thereof; polythiophene and derivatives thereof; polypyrrole and derivatives thereof, polyphenylenevinylene and derivatives thereof; polythienylenevinylene and derivatives thereof; and polyfluorene and derivatives thereof.

From the viewpoint of the conversion efficiency, the electron-donating compound used in the organic photoelectric conversion element is preferably a polymer compound having a repeating unit selected from the group consisting of the repeating units represented by the following formula (10) and the following formula (11), and is more preferably a polymer compound having a repeating unit represented by the following formula (10). 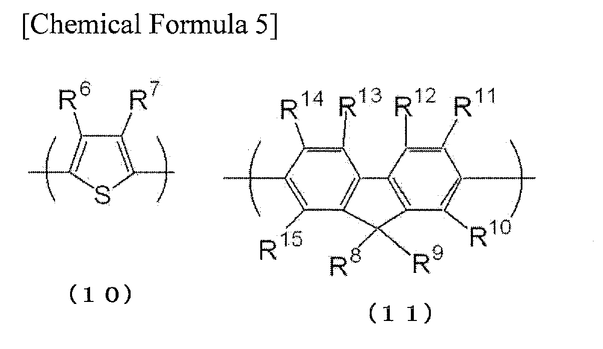 In formulas (10) and (11), R⁶, R⁷, R⁸, R⁹, R¹⁰, R¹¹, R¹², R¹³, R¹⁴ and R¹⁵ are the same as or different from each other, and each represent a hydrogen atom, an alkyl group, an alkoxy group or an aryl group.

In formula (10), specific examples of R⁶ and R⁷, in the case where R⁶ and R⁷ are alkyl groups, include the same alkyl groups as the above-listed examples of the alkyl groups for R¹. Specific examples of R⁶ and R⁷, in the case where R⁶ and R⁷ are alkoxy groups, include the same alkoxy groups as the above-listed examples of the alkoxy groups for R¹. Specific examples of R⁶ and R⁷, in the case where R⁶ and R⁷ are aryl groups, include the same aryl groups as the above-listed examples of the aryl groups for R¹.

In formula (10), from the viewpoint of the conversion efficiency, at least one of R⁶ and R⁷ is preferably an alkyl group having 1 to 20 carbon atoms and more preferably an alkyl group having 4 to 8 carbon atoms.

In formula (11), specific examples of R⁸ to R¹⁵, in the case where R⁸ to R¹⁵ are alkyl groups, include the same alkyl groups as the above-listed examples of the alkyl groups for R¹. Specific examples of R⁸ to R¹⁵, in the case where R⁸ to R¹⁵ are alkoxy groups, include the same alkoxy groups as the above-listed examples of the alkoxy groups for R¹. Specific examples of R⁸ to R¹⁵, in the case where R⁸ to R¹⁵ are aryl groups, include the same aryl groups as the above-listed examples of the aryl groups for R¹ .

In formula (11), from the viewpoint of the easiness in synthesizing the monomer, R¹⁰ to R¹⁵ are each preferably a hydrogen atom. Also from the viewpoint of the conversion efficiency, R⁸ and R⁹ are each preferably an alkyl group having 1 to 20 carbon atoms or an aryl group having 6 to 20 carbon atoms, and more preferably an alkyl group having 5 to 8 carbon atoms or an aryl group having 6 to 15 carbon atoms.

The amount of fullerene derivative included in the composition of the present invention is preferably 10 to 1000 parts by weight and more preferably 50 to 500 parts by weight in relation to 100 parts by weight of the electron-donating compound.

### <Organic Photoelectric Conversion Element>

The organic photoelectric conversion element of the present invention comprises a pair of electrodes, at least one of the electrodes being transparent or translucent, and an organic layer disposed between the pair of electrodes, wherein the organic layer includes the fullerene derivative of the present invention or the composition of the present invention. By having such features, the organic photoelectric conversion element of the present invention has a high open-end voltage.

The pair of electrodes correspond to an anode and a cathode, respectively. Specifically, the organic photoelectric conversion element of the present invention has an anode, a cathode and a layer comprising the fullerene derivative or the composition, disposed between the anode and the cathode.

The fullerene derivative of the present invention may be used either as an electron-accepting compound or as an electron-donating compound, but preferably used as an electron-accepting compound.

Next, the operation mechanism of the organic photoelectric conversion element is described. Photoenergy incident from the transparent or translucent electrode is absorbed by the electron-accepting compound and/or the electron-donating compound to generate excitons each composed of an electron and a hole bound to each other. When the generated excitons move and reach the heterojunction interface where the electron-accepting compound and the electron-donating compound are adjacent to each other, electrons and holes separate, due to the difference between the HOMO energy and the LUMO energy in each of these compounds at the interface, to generate charges (electrons and holes) that can move independently. The generated charges move to the electrodes respectively, and hence the charges can be taken out as electrical energy (current) to the outside.

As the specific example of the organic photoelectric conversion element of the present invention, either of the following is preferable:
1. an organic photoelectric conversion element comprising a pair of electrodes, at least one of the electrodes being transparent or translucent, a first layer disposed between the electrodes and including the fullerene derivative of the present invention as an electron-accepting compound, and a second layer disposed adjacent to the first layer and including an electron-donating compound; and
2. an organic photoelectric conversion element comprising a pair of electrodes, at least one of the electrodes being transparent or translucent, and at least one layer disposed between the electrodes and including the fullerene derivative of the present invention as an electron-accepting compound and an electron-donating compound.

From the viewpoint of including many heterojunction interfaces, the above 2. organic photoelectric conversion element is preferable. Also, in the organic photoelectric conversion element of the present invention, an additional layer may be disposed between at least one of the electrodes and the layer including the fullerene derivative used in the present invention. Examples of the additional layer include a charge transport layer which transport holes or electrons.

In the above 2. organic photoelectric conversion element, the proportion of the fullerene derivative in the organic layer including the fullerene derivative and the electron-donating compound is preferably 10 to 1000 parts by weight and more preferably 50 to 500 parts by weight in relation to 100 parts by weight of the electron-donating compound.

In the above 2. organic photoelectric conversion element, the organic layer including the fullerene derivative and the electron-donating compound can be produced by using a composition comprising the fullerene derivative and the electron-donating compound.

The layer including the fullerene derivative used in the organic photoelectric conversion element of the present invention is preferably formed of an organic thin film comprising the fullerene derivative. The thickness of the organic thin film is usually 1 nm to 100 µm, preferably 2 nm to 1000 nm, more preferably 5 nm to 500 nm, and furthermore preferably 20 nm to 200 nm.

The organic photoelectric conversion element of the present invention is usually formed on a substrate. The substrate has only to be a substrate that does not undergo any change when electrodes are formed and a layer of an organic substance is formed. Examples of the material of the substrate include glass, plastic, polymer film, and silicon. In the case of an opaque substrate, the opposite electrode (that is, the electrode far from the substrate) is preferably transparent or translucent.

Examples of the material of the transparent or translucent electrode include a conductive metal oxide film and a translucent metal thin film. Specifically, used as the electrode materials are the films (such as NESA) prepared by using conductive materials composed of, for example, indium oxide, zinc oxide, tin oxide, and indium tin oxide (ITO) and indium zinc oxide, which are composite materials of these; also used are gold, platinum, silver, copper and the like; preferable are ITO, indium zinc oxide, tin oxide and the like. Examples of the method for preparing the electrode include a vacuum deposition method, a sputtering method, an ion plating method, and a plating method. As the electrode materials, organic transparent conductive films of, for example, polyaniline and derivatives thereof, and polythiophene and derivatives thereof may also be used.

Further, it is possible to use metals, conductive polymers and the like as the electrode materials, and one electrode of the pair of electrodes is preferably of a material having a small work function. The materials used as such electrode materials are, for example, as follows: metals such as lithium, sodium, potassium, rubidium, cesium, magnesium, calcium, strontium, barium, aluminum, scandium, vanadium, zinc, yttrium, indium, cerium, samarium, europium, terbium and ytterbium; alloys of two or more of these metals; alloys of one or more of these metals and one or more of gold, silver, platinum, copper, manganese, titanium, cobalt, nickel, tungsten and tin; graphite; or graphite intercalation compounds.

Examples of the alloys include a magnesium-silver alloy, a magnesium-indium alloy, a magnesium-aluminum alloy, an indium-silver alloy, a lithium-aluminum alloy, a lithium-magnesium alloy, a lithium-indium alloy and a calcium-aluminum alloy.

As the material used as a buffer layer as an additional layer, halides and oxides of alkali metals and alkali earth metals such as lithium fluoride can be used. Fine particles of an inorganic semiconductor such as titanium oxide can also be used.

### <Method for Producing Organic Thin Film>

The method for producing the organic thin film is not particularly limited, and examples of such a method include a method for forming a film from a solution containing the fullerene derivative used in the present invention.

The solvent used for the film formation from a solution is not particularly limited as long as the solvent dissolves the fullerene derivative used in the present invention. Examples of such a solvent include: hydrocarbon solvents such as toluene, xylene, mesitylene, tetralin, decalin, bicyclohexyl, n-butylbenzene, s-butylbenzene and t-butylbenzene; halogenated saturated hydrocarbon solvents such as carbon tetrachloride, chloroform, dichloromethane, dichloroethane, chlorobutane, bromobutane, chloropentane, bromopentane, chlorohexane, bromohexane, chlorocyclohexane and bromocyclohexane; halogenated unsaturated hydrocarbon solvents such as chlorobenzene, dichlorobenzene and trichlorobenzene; and ether solvents such as tetrahydrofuran and tetrahydropyran. Usually, the fullerene derivative can be dissolved in the above-listed solvents in a content of 0.1 % by weight or more.

The solution may further contain a polymer compound. Specific examples of the solvent used for the solution include the above listed solvents; however, from the viewpoint of the solubility of the polymer compound, aromatic hydrocarbon solvents are preferable, and toluene, xylene and mesitylene are more preferable.

For the film formation from a solution, for example, the following coating methods can be used: spin coating, casting, microgravure coating, gravure coating, bar coating, roll coating, wire bar coating, dip coating, spray coating, screen printing, flexographic printing, offset printing, ink jet printing, dispenser printing, nozzle coating and capillary coating; and spin coating, flexographic printing, ink jet printing and dispenser printing are preferable.

The organic photoelectric conversion element generates photovoltaic power between the electrodes by the irradiation from the transparent or translucent electrode with light such as sunlight, and thus may be operated as an organic thin film solar cell. It may be also used as an organic thin film solar cell module by integrating a plurality of organic thin film solar cells.

Also it leads photocurrent by the irradiation from the transparent or translucent electrode with light under the condition that a voltage is applied between the electrodes, and thus may be operated as an organic optical sensor. It may be also used as an organic image sensor by integrating a plurality of organic optical sensors.

### Examples

Hereinafter, Examples are presented for the purpose of describing the present invention in further detail, but the present invention is not limited to these Examples.

As the reagents and the solvents used in the syntheses, commercial products were used as received or commercial products purified by distillation in the presence of a desiccant were used. As C₆₀ fullerene, the product manufactured by Frontier Carbon Corp. was used. NMR spectra were measured with "MH500" (trade name) manufactured by JEOL Lid., and tetramethylsilane (TMS) was used as internal standard. Infrared absorption spectra were measured with "FT-IR 8000" (trade name) manufactured by Shimadzu Corp.. MALDI-TOF MS spectra were measured with "AutoFLEX-T2" (trade name) manufactured by Bruker Corp.

### (Synthesis Example 1)

### Synthesis of benzyl [2-(2-hydroxyethoxy)ethylamino]acetate

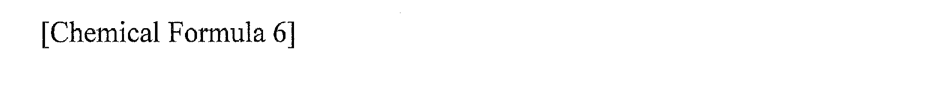

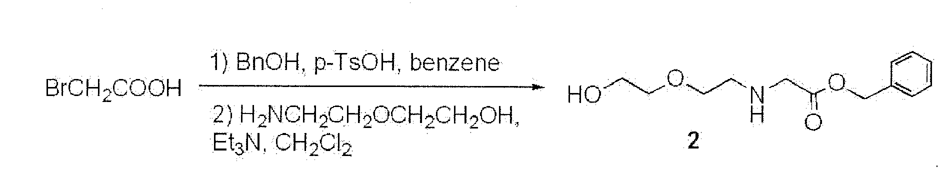

[First Step]: Bromoacetic acid (20.8 g, 150 mmol), benzyl alcohol (16.2 g, 150 mmol), p-toluenesulfonic acid (258 mg, 1.5 mmol) and benzene (300 mL) were added to a two-necked flask equipped with a Dean-Stark trap, and were subjected to dehydration condensation at 120°C for 24 hours, and then the solvent was distilled off under reduced pressure with an evaporator. Then, the residue was purified by silica gel flash column chromatography (hexane/ethyl acetate=10/1, 5/1), and thus bromoacetic acid benzyl ester (34.3 g, 150 mmol) was obtained quantitatively as a yellow oily product.

R_{f} 0.71 (hexane/ethyl acetate=4/1);
¹H NMR (500 MHz, ppm, CDCl₃, J=Hz) δ 3.81 (s, 2H), 5.14 (s, 2H), 7.31 (s, 5H);
¹³C NMR (125 MHz, ppm, CDCl₃) δ 25.74, 67.79, 128.27, 128.48, 128.54, 134.88, 166.91;
IR (neat, cm⁻¹) 2959, 1751, 1458, 1412, 1377, 1167, 972, 750, 698

[Second Step]: In an argon atmosphere, triethylamine (17 mL, 120 mmol) was added to a dichloromethane (90 mL) solution of the above bromoacetic acid benzyl ester (13.7 g, 60 mmol) at 0°C, and the obtained mixed solution was stirred at the same temperature for 20 minutes. Then, a dichloromethane (40 mL) solution of 2-(2-aminoethoxy)ethanol (12 mL, 120 mmol) was added and stirred at room temperature for 4 hours. Then, the organic layer of the obtained reaction solution was washed with water three times, and then dried with anhydrous magnesium sulfate, and the solvent was distilled off under reduced pressure with an evaporator. Then, the residue was purified by silica gel flash column chromatography (developing solvent:ethyl acetate/methanol=1/0, 10/1, 5/1) and thus benzyl [2-(2-hydroxyethoxy)ethylamino]acetate, which is a glycine ester, (hereinafter, referred to as "glycine ester 2") (12.2 g, 48.0 mmol) was obtained as a colorless oily product, with a yield of 80%.

R_{f} 0.48 (ethyl acetate/methanol=2/1);
¹H NMR (500 MHz, ppm, CDCl₃, J=Hz) δ 2.83 (t, 2H, J=5.1 Hz), 3.50 (s, 2H), 3.52 (t, 2H, J=4.6 Hz), 3.58 (t, 2H, J=5.0 Hz), 3.65 (t, 2H, J=4.6 Hz), 5.11 (s, 2H), 7.28-7.30 (m, 5H);
¹³C NMR (125 MHz, ppm, CDCl₃) δ 48.46, 50.25, 61.29, 66.38, 69.80, 72.23, 126.63, 128.12, 128.37, 135.30, 171.78;
IR (neat, cm⁻¹) 3412, 2880, 1719, 1638, 1560, 1508, 1458, 1067, 669

### (Synthesis Example 2)

### Synthesis of [2-(2-methoxyethoxy)ethylamino] acetic acid

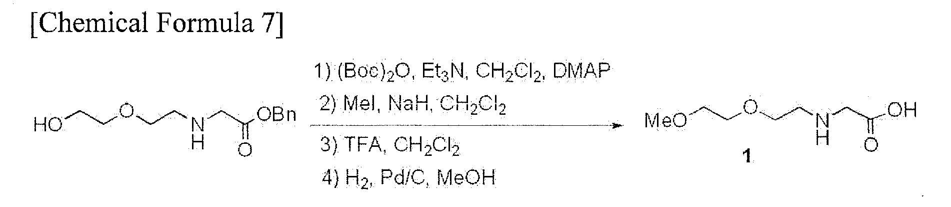

[First Step]: In an argon atmosphere, triethylamine (4.3 mL, 31 mmol) was added to a dichloromethane (50 mL) solution of glycine ester 2 (6.58 g, 26 mmol) at 0°C, and then, 4-(N,N-dimethylamino)pyridine (DMAP) (32 mg, 0.26 mmol) was added. The obtained mixed solution was stirred for 20 minutes, and then, a dichloromethane (10 mL) solution of di-tert-butyl dicarbonate (6.77 g, 31 mmol) was added dropwise to the mixed solution. The reaction mixed solution was stirred at room temperature for 4 hours, then poured into an Erlenmeyer flask containing water to terminate the reaction, and then the obtained mixture was extracted with diethyl ether three times. The organic layer was dried, then concentrated under reduced pressure, and then purified by silica gel flash column chromatography (developing solvent: hexane/ethyl acetate=3/1, 2.5/1, 2/1), and thus benzyl {tert-butoxycarbonyl-[2-(2-hydroxy-ethoxy)ethyl]amino}acetate (5.83 g, 16.5 mmol) was obtained as a colorless oily product with a yield of 63%.

R_{f} 0.58 (ethyl acetate/methanol=20/1);
¹H NMR (500 MHz, ppm, CDCl₃, J=Hz) δ 1.34 (d, 9H, J=54.5 Hz), 2.19 (brs, 1H), 3.38-3.45 (m, 4H), 3.50-3.60 (m, 4H), 3.99 (d, 2H, J=41.3 Hz), 5.09 (d, 2H, J=4.1 Hz), 7.25-7.30 (m, 5H);
¹³C NMR (125 MHz, ppm, CDCl₃) δ 27.82, 28.05, 47.90, 48.20, 49.81, 50.39, 61.23, 66.42, 69.92, 72.12, 80.08, 127.93, 128.14, 135.25, 154.99, 155.19, 169.94, 170.07;
IR (neat, cm⁻¹ 3449, 2934, 2872, 1751, 1701, 1458, 1400, 1367, 1252, 1143;
Anal. Calcd for C₁₈H₂₇NO₆: C, 61.17; H, 7.70; N, 3.96. Found: C, 60.01; H, 7.75; N, 4.13

[Second Step]: In an argon gas atmosphere, a tetrahydrofuran (THF) (10 mL) solution of benzyl {tert-butoxycarbonyl-[2-(2-hydroxy-ethoxy)ethyl]amino}acetate (5.83 g, 16.5 mmol) was added dropwise to a THF (10 mL) solution of sodium hydride (1.2 g, 24.8 mmol, 50% in mineral oil) at 0°C, and the obtained mixture was stirred at the same temperature for 20 minutes, and then, iodomethane (1.6 mL, 24.8 mmol) was added at 0°C to the mixture. The obtained reaction mixed solution was stirred at room temperature for 20 hours, and then, water was added to the reaction mixed solution to terminate the reaction, while the reaction mixed solution was being cooled in an ice bath, and then the obtained mixture was extracted with diethyl ether three times. The organic layer was dried, then concentrated under reduced pressure, and purified by silica gel flash column chromatography (developing solvent: hexane/ethyl acetate=5/1, 3/1), and thus benzyl {tert-butoxycarbonyl-[2-(2-methoxy-ethoxy)ethyl]amino}acetate (3.02 g, 8.21 mmol) was obtained as a colorless oily product, with a yield of 50%.

R_{f} 0.54 (hexane/ethyl acetate=1/1);
¹H NMR (500 MHz, ppm, CDCl₃, J=Hz) δ 1.34 (d, 9H, J=51.8 Hz), 3.28 (d, 3H, J=2.7 Hz), 3.37-3.46 (m, 6H), 3.52 (dt, 2H, J=5.4 Hz, 16.5 Hz), 4.02 (d, 2H, J=34.8 Hz), 5.09 (d, 2H, J=4.5 Hz), 7.24-7.30 (m, 5H); ¹³C NMR (125 MHz, ppm, CDCl₃) δ 24.93, 25.16, 44.68, 45.00, 46.70, 47.40, 55.78, 63.30, 67.22, 68.60, 76.95, 124.98, 125.14, 125.36, 132.49, 151.99, 152.31, 166.84, 166.96;
IR (neat, cm⁻¹) 2880 1751, 1701, 1560, 1458, 1400, 1366, 1117, 698, 617;
Anal. Calcd for C₁₉H₂₉NO₆: C, 62.11; H, 7.96; N, 3.81. Found: C, 62.15; H, 8.16; N, 3.83

[Third Step]: In an argon atmosphere, trifluoroacetic acid (TFA) (9.0 mL) was added to a dichloromethane (17 mL) solution of benzyl {tert-butoxycarbonyl-[2-(2-methoxy-ethoxy)ethyl]amino}acetate (3.02 g, 8.21 mmol), and the obtained mixture was stirred at room temperature for 7 hours. Then, a 10% aqueous solution of sodium carbonate was added to the mixture to adjust the pH of the mixture to 10, the obtained mixture was extracted with dichloromethane, and the obtained organic layer was dried with anhydrous magnesium sulfate and concentrated under reduced pressure, and thus benzyl [2-(2-methoxy-ethoxy)ethylamino]acetate (2.18 g, 8.19 mmol) was obtained quantitatively as a yellow oily product.

R_{f} 0.32 (ethyl acetate/methanol=20/1);
¹H NMR (500 MHz, ppm, CDCl₃, J=Hz) δ 1.99 (brs, 1H), 2.83 (t, 2H, J=5.3 Hz), 3.38 (s, 3H), 3.50 (s, 2H), 3.54 (t, 2H, J=4.6 Hz), 3.60-3.62 (m, 4H), 5.17 (s, 2H), 7.32-7.38 (m, 5H);
¹³C NMR (125 MHz, ppm, CDCl₃) δ 48.46, 50.66, 58.76, 66.20, 70.00, 70.44, 71.64, 128.09, 128.33, 135.44, 171.84;
IR (neat, cm⁻¹) 3350, 2876, 1736, 1560, 1458, 1117, 1030, 698, 619; Anal. Calcd for C₁₄H₂₁NO₄: C, 62.90; H, 7.92; N, 5.24. Found: C, 62.28; H, 8.20; N, 5.05

[Fourth Step]: Activated carbon (219 mg) having been made to support 10% by weight of palladium was added to a methanol (27 mL) solution of benzyl [2-(2-methoxy-ethoxy)ethylamino]acetate (2.19 g, 8.19 mmol) at room temperature, the obtained reaction mixture was purged with hydrogen gas, and then, the reaction mixture was stirred in a hydrogen atmosphere at room temperature for 7 hours. Pd/C was removed with a glass filter over which a celite pad was spread, and the celite layer was washed with methanol, the filtrate was concentrated under reduced pressure, and thus [2-(2-methoxyethoxy)ethylamino]acetic acid (hereinafter, referred to as "glycine derivative 1") (1.38 g, 7.78 mmol), which is a glycine derivative, was obtained as a yellow oily product, with a yield of 95%.

¹H NMR (500 MHz, ppm, MeOD, J=Hz) δ 3.21 (t, 2H, J=5.1 Hz), 3.38 (s, 3H), 3.51 (s, 2H), 3.57 (t, 2H, J=4.4 Hz), 3.65 (t, 2H, J=4.6 Hz), 3.73 (t, 2H, J=5.1 Hz);
¹³C NMR (125 MHz, ppm, MeOD) δ 48.13, 50.49, 59.16, 67.08, 71.05, 72.85, 71.10;
IR (neat, cm⁻¹) 3414, 2827, 1751, 1630, 1369, 1111, 1028, 851, 799; Anal. Calcd for C₇H₁₅NO₄: C, 47.45; H, 8.53; N, 7.90. Found: C, 46.20; H, 8.49; N, 7.43

### Example 1

### (Synthesis of Fullerene Derivative A)

### N-Methoxyethoxyethyl-2-(2,2-bithiophene)fulleropyrrolidine (KM-BT)

By the 1,3-dipolar cycloaddition reaction (the Prato reaction) between fullerene and the iminium cation produced by the decarboxylation of the imine produced by the reaction between the glycine derivative 1 and 2-thienyl-2-thiophenecarboaldehyde, N-methoxyethoxyethyl-2-(2,2-bithiophene)fulleropyrrolidine (KM-BT) was synthesized. Hereinafter, this is referred to as the "fullerene derivative A."

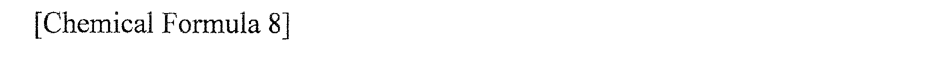

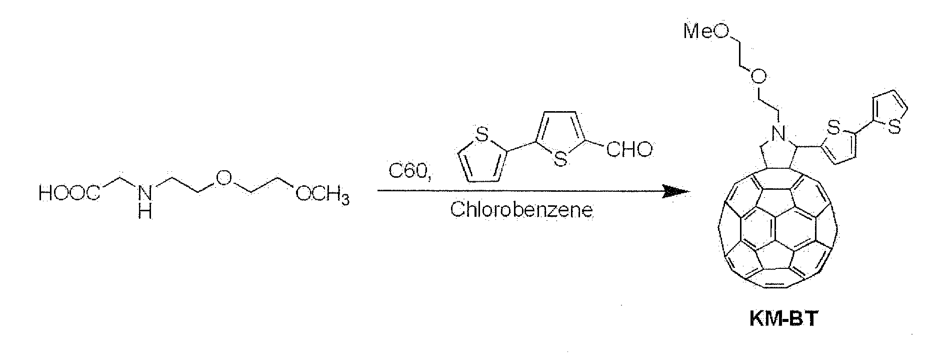

In a two-neck flask (200 mL) equipped with a Dimroth condenser, fullerene C₆₀ (250 mg, 0.35 mmol), the glycine derivative 1 (92 mg, 0.52 mmol) and 2-thienyl-2-thiophenecarbaldhyde (135 mg, 0.69 mmol) synthesized by the method described in The Journal of Organic Chemistry, 2007, 72, 2631-2643 were placed, then chlorobenzene (50 mL) was added in the flask, and the obtained mixture was heated under refluxing for 2 hours. The obtained reaction solution was allowed to cool to room temperature, then the solvent was removed with a rotary evaporator, the residue was purified by silica gel flash column chromatography (carbon disulfide, toluene/ethyl acetate=50/1), and thus 159 mg (0.15 mmol, yield: 44%) of a brown powder was collected. The powder was washed with methanol five times and then dried under reduced pressure, and thus the fullerene derivative A (KM-BT) was obtained.

Rf=0.78 (toluene/ethyl acetate=5/1)
¹H NMR (500 MHz, ppm, CDCl₃, J=Hz) δ 2.94-3.01 (1H, m), 3.42 (3H, s), 3.60-3.69 (3H, m), 3.76-3.83 (2H, m), 3.99-4.03 (1H, m), 4.06-4.13 (1H, m), 4.29 (1H, d, J=9.7 Hz), 5.20 (1H, d, J=9.6 Hz), 5.44 (1H, s), 6.98 (1H, dd, J=5.5 Hz, 3.7 Hz), 7.07 (1H, d, J=3.7 Hz), 7.15 (1H, dd, J=3.7 Hz, 1.0 Hz), 7.18 (1H, dd, J=5.1 Hz, 1.0 Hz, 7.28 (1H, d, J=3.7 Hz);
¹³C NMR (125 MHz, ppm, CDCl₃) δ 52.35, 59.04, 67.74, 68.87, 68.90, 70.52, 70.55, 71.99, 76.03, 76.05, 78.08, 122.89, 123.78, 124.50, 127.77, 128.90, 135.44, 135.82, 136.53, 137.08, 137.31, 138.66, 139.66, 139.87, 139.94, 139.96, 140.07, 141.52, 141.59, 141.80, 141.87, 141.95, 142.04, 142.07, 142.14, 142.20, 142.49, 142.60, 142.89, 143.06, 144.29, 144.54, 144.63, 145.06, 145.15, 145.20, 145.24, 145.35, 145.40, 145.43, 145.52, 145.67, 145.84, 145.98, 146.03, 146.08, 146.10, 146.22, 146.73, 147.22, 153.05, 154.01, 156.02;
IR (Neat, cm⁻¹) 2864, 1636, 1541, 1458, 1424, 1339, 1197, 1180, 1107, 1044, 839, 795, 767, 727, 691, 575, 552, 527;
MALDI-TOF-MS (matrix: SA) found 1029.0873 (calcd for C₇₅H₁₉NO₂S₂ Exact Mass: 1029.0857).

### Example 2

### (Preparation of Organic Thin Film Solar Cell)

As an electron-donating compound, regioregular poly(3-hexylthiophene) (Lot No.: 09007KH, manufactured by Aldrich Corp.) was dissolved in chlorobenzene in a concentration of 1 % by weight. In the obtained solution, the fullerene derivative A, which is an electron-accepting compound, was added and mixed in an amount equivalent to the amount of the electron-donating compound. Then, the obtained mixture was filtered with a Teflon (trade mark) filter of 1.0 µm in pore size, and thus a coating solution was prepared.

A glass substrate on which an ITO film with a thickness of 150 nm was provided by sputtering was subjected to ozone-UV treatment to perform surface treatment. Next, on the obtained glass substrate having been surface-treated, the coating solution was spin-coated, and thus an organic layer (active layer) (film thickness: about 100 nm) of an organic thin film solar cell was obtained. Then, the coated substrate was baked under the conditions of 90°C under vacuum for 60 minutes. Then, on the organic layer, with a vacuum deposition apparatus, lithium fluoride was vapor-deposited in a thickness of 4 nm, and then, aluminum was vapor-deposited in a thickness of 100 nm (degree of vacuum: 1 to 9×10⁻³ Pa in both of these depositions), and thus an organic thin film solar cell (shape: 2 mm x 2 mm square) was prepared.

### Example 3

### (Synthesis of Fullerene Derivative B)

### N-Methoxyethoxyethyl-2-(2,2-bithiophene) C₇₀ fulleropyrrolidine (KM-BT70)

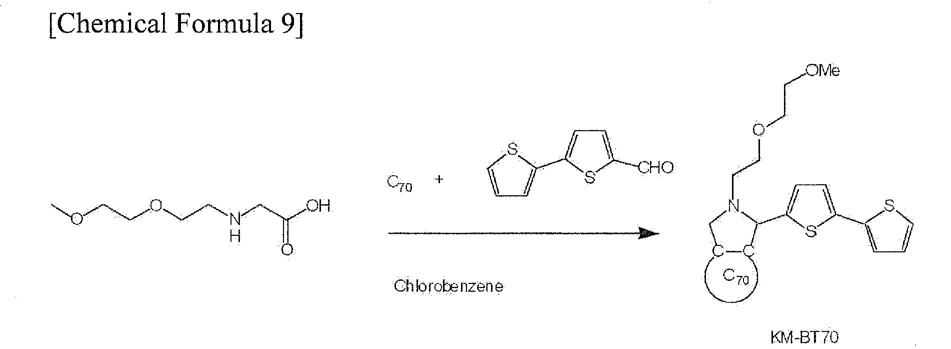

Chlorobenzene (50 mL) was added to fullerene C₇₀ (250 mg, 0.30 mmol), the glycine derivative 1 (79 mg, 0.45 mmol) and 2-thienyl-2-thiophenecarbaldehdye, and the obtained mixture was heated under refluxing at 120°C for 2 hours. The mixture was allowed to cool at room temperature, then the mixture was concentrated under reduced pressure to distill off the solvent, the obtained residue was purified by silica gel flash column chromatography (carbon disulfide, toluene/ethyl acetate=1/0 to 50/1), and thus 139 mg (0.12 mmol, yield: 41%) of a brown powder was collected. The powder was washed with methanol five times, and then dried under vacuum, and thus N-methoxyethoxyethyl-2-(2,2-bithiophene) C₇₀ fulleropyrrolidine (KM-BT70), which is a fullerene derivative, was obtained. Hereinafter, this is referred to as the "fullerene derivative B."

Rf=0.78 (toluene/ethyl acetate=5/1)
¹H NMR (500 MHz, ppm, CDCl₃, J=Hz) δ 2.49-2.58 (1H, m), 2.64-2.70 (1H, m), 3.23 (1H, d, J=10.0 Hz), 3.32 (3H, s), 3.37 (3H, s), 3.39 (3H, s), 3.43 (1H, d, J=10.0 Hz), 3.48 (3H, s), 3.55-3.60 (2H, m), 3.63 (1H, d, J=10.1 Hz), 3.66-3.70 (2H, m), 3.77 (1H, t, J=4.8 Hz), 3.79-3.88 (2H, m), 3.91-3.96 (2H, m), 4.29 (1H, d, J=10.1 Hz), 4.36 (1H, s), 4.43 (1H, d, J=10.1 Hz), 4.48 (1H, d, J=10.1 Hz), 4.55 (1H, s), 4.73 (1H, d, J=13.8 Hz), 4.91 (1H, d, J=9.6 Hz), 6.90-6.95 (m), 3.49 (dd, J=3.6 Hz, 1.0 Hz), 7.01 (d, J=3.7 Hz), 7.03-7.08 (m), 7.12 (dd, J=5.0 Hz, 9.0 Hz), 7.15 (d, J=4.6 Hz), 7.23 (s), 7.24 (d, J=1.9 Hz), 7.27-7.29 (m), 7.45 (d, J=3.7 Hz);
¹³C NMR (125 MHz ppm, CDCl₃ δ 51.74 51.77, 51.82, 58.98, 59.05, 59.11, 59.17, 60.30, 60.33, 61.95, 65.81, 67.85, 69.08, 69.86, 69.86, 70.20, 70.30, 70.41, 70.44, 70.52, 71.84, 71.93, 71.96, 72.03, 74.81, 77.60, 77.74, 78.08, 78.47, 122.56, 123.22, 123.69, 123.75, 123.78, 123.88, 123.94, 124.49, 124.52, 124.59, 124.64, 127.71, 127.74, 127.86, 127.90, 128.99, 129.14, 129.28, 131.16, 131.19, 131.25, 131.43, 131.57, 131.99, 133.55, 133.79, 137.17, 137.71, 138.63, 139.62, 139.86, 140.53, 140.75, 142.01, 142.69, 142.78, 143.12, 143.20, 143.39, 144.50, 144.53, 145.29, 145.42, 145.68, 145.74, 146.16, 146.22, 146.53, 146.61, 146.75, 146.78, 146.84, 146.87, 146.90, 146.97, 147.00, 147.03, 147.07, 147.36, 147.39, 147.63, 147.66, 147.97, 148.02, 148.19, 148.34, 148.65, 148.77, 148.93, 149.03, 149.09, 149.11, 149.14, 149.19, 149.22, 149.30, 149.35, 149.70, 149.74, 149.85, 150.25, 150.52, 150.59, 150.70, 150.79, 151.03, 151.28, 151.42;
MALDI-TOF-MS (matrix: SA) found 1149.1051 (calcd for C₈₅H₁₉NO₂S₂ Exact Mass: 1149.0857)

### Example 4

### (Preparation of Organic Thin Film Solar Cell)

An organic thin film solar cell was prepared by performing the same operations as in Example 2 except that the coating solution was obtained by using the fullerene derivative B in place of the fullerene derivative A.

### Example 5

### (Synthesis of Fullerene Derivative C)

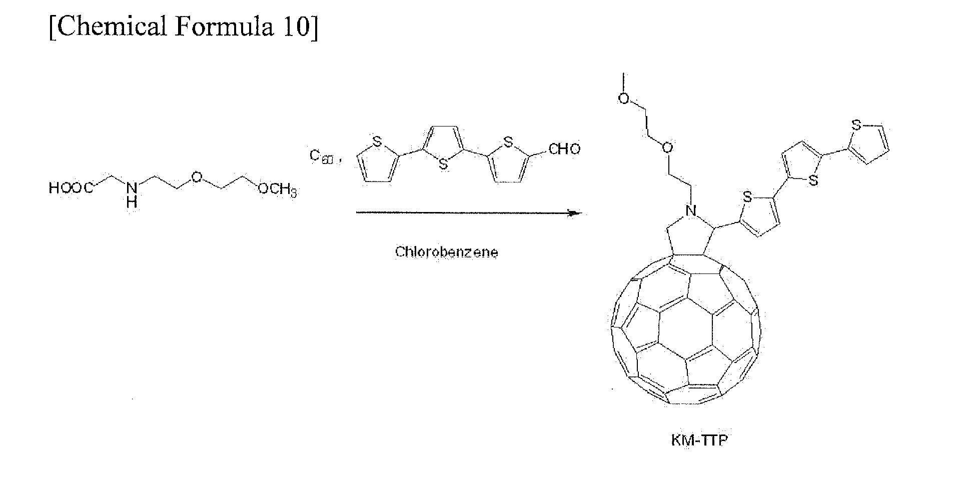

In a two-neck flask (100 mL) equipped with a Dimroth condenser, fullerene C₆₀ (250 mg, 0.35 mmol), the glycine derivative 1 (92 mg, 0.52 mmol) and 2,2':5',2"-terthiophene-5-carbaldhyde (192 mg, 0.694 mmol) were added, then chlorobenzene (50 mL) was added in the flask, and the obtained mixture was heated under refluxing for 3 hours. The obtained reaction solution was allowed to cool to room temperature, then the solvent was removed with a rotary evaporator, the residue was purified by silica gel flash column chromatography (carbon disulfide, toluene/ethyl acetate=30/1 to 20/1) and successively purified by preparative thin layer chromatography (carbon disulfide/ethyl acetate=10/1), and thus 157 mg (0.14 mmol, yield: 41%) of a brown powder was collected. The powder was washed with methanol five times and then dried under reduced pressure, and thus N-methoxyethoxyethyl-2-(2,2':5',2"-terthiophene)fulleropyrrolidine (KM-TPP), which is a fullerene derivative, was obtained. Hereinafter, this is referred to as the "fullerene derivative C."

¹H NMR (500 MHz, ppm, CDCl₃, J=Hz) δ 2.92-2.96 (1H, m), 3.39 (3H, s), 3.57-3.66 (3H, m), 3.73-3.80 (2H, m), 3.95-3.99 (1H, m), 4.04-4.08 (1H, m), 4.26 (1H, d, J=9.6 Hz), 5.20 (1H, d, J=10.1 Hz), 5.42 (1H, s), 6.96 (1H, dd, j=5.1 Hz, 3.6 Hz), 7.00 (2H, dd, J=9.2 Hz, 3.6 Hz), 7.03 (d, 1H, J=3.6 Hz), 7.09 (1H, dd, J=3.4 Hz, 1.0 Hz), 7.15 (1H, dd, J=5.0 Hz, 0.9 Hz), 7.26 (1H, d, J=3.7 Hz);
¹³C NMR (125 MHz, ppm, CDCl₃) δ52.40, 58.81, 67.68, 68.67, 70.49, 71.93, 75.83, 77.94, 122.71, 123.61, 124.17, 124.27, 124.44, 127.69, 128.74, 135.27, 135.65, 135.92, 136.35, 136.84, 136.94, 138.21, 139.54, 139.74, 139.93, 140.03, 141.37, 141.43, 141.62, 141.70, 141.79, 141.88, 141.98, 142.01, 142.33, 142.45, 142.74, 142.90, 144.12, 144.40, 144.46, 144.89, 144.97, 145.03, 145.13, 145.18, 145.21, 145.29, 145.39, 145.50, 145.67, 145.81, 145.87, 145.94, 146.01, 146.08, 146.54, 147.03, 152.71, 152.81, 153.77, 155.79;
IR (Neat, cm⁻¹) 2915, 2866, 2807, 1634, 1510, 1462, 1424, 1180, 1107, 1044, 793, 727, 691, 527;
MALDI-TOF-MS (matrix: SA) found 1111.0753 (calcd for C₇₉H₂₁NO₂S₃ Exact Mass: 1111.0734).

### Example 6

### (Preparation of Organic Thin Film Solar Cell)

An organic thin film solar cell was prepared by performing the same operations as in Example 2 except that the coating solution was obtained by using the fullerene derivative C in place of the fullerene derivative A.

### Comparative Example 1

### (Preparation of Organic Thin Film Solar Cell)

An organic thin film solar cell was prepared by performing the same operations as in Example 2 except that the coating solution was obtained by using [60]PCBM (Phenyl C61-butyric acid methyl ester, trade name: "E100," Lot No.:8A0125A, manufactured by Frontier Carbon Corp.) in place of the fullerene derivative A.

### (Evaluation of Organic Thin Film Solar Cells)

The photoelectric conversion efficiency of each of the organic thin film solar cells prepared in Examples and Comparative Example was obtained by measuring the current and voltage generated by irradiating each of the organic thin film solar cells with a predetermined amount of light by using a solar simulator (trade name: "OTENTO-SUN II," AM 1.5G filter, irradiance: 100 mW/cm², manufactured by Bunkoukeiki Co., Ltd.). The results thus obtained are shown in Table 1.

**[Table 1]**

| | Fullerene derivative | Photoelectric conversion efficiency (%) |
|---|---|---|
| Example 2 | Fullerene derivative A | 2.5 |
| Example 4 | Fullerene derivative B | 2.5 |
| Example 6 | Fullerene derivative C | 2.35 |
| Comparative Example 1 | [60]PCBM | 1.9 |

## Claims

1. A fullerene derivative represented by the following formulas (1). 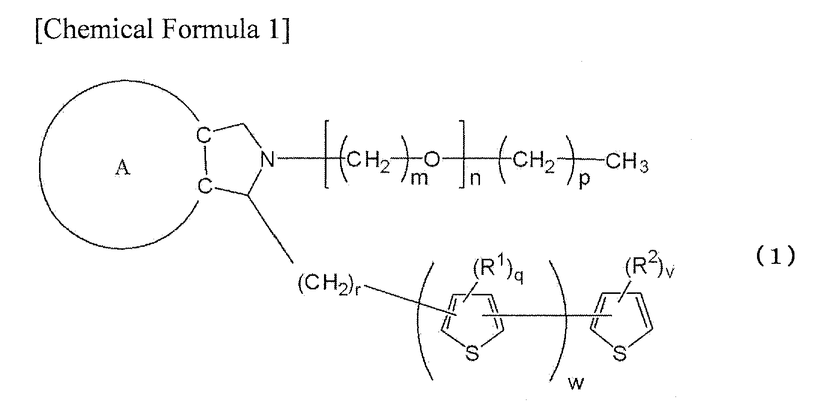 [In formula (1), a ring A represents a fullerene skeleton having 60 or more carbon atoms; R¹ and R² are the same as or different from each other, and each represent a halogen atom, an alkyl group, an alkoxy group or an aryl group (wherein the whole or a part of hydrogen atoms belonging to the alkyl group, the alkoxy group or the aryl group may be substituted with halogen atoms or a halogen atom); m represents an integer of 1 to 6, n represents an integer of 1 to 4, p represents an integer of 0 to 5, w represents an integer of 1 to 6, r represents an integer of 0 to 4, q represents an integer of 0 to 2 and v represents an integer of 0 to 3; when there are a plurality of R¹s, the R¹s may be the same as or different from each other; when there are a plurality of R²s, the R²s may be the same as or different from each other; when there are a plurality of m's, the m's may be the same as or different from each other; and when there are a plurality of q's, the q's may be the same as or different from each other]

2. A composition comprising the fullerene derivative according to claim 1 and an electron-donating compound.

3. The composition according to claim 2, wherein the electron-donating compound is a polymer compound.

4. An organic photoelectric conversion element comprising a pair of electrodes, at least one of the electrodes being transparent or translucent, and an organic layer disposed between the pair of electrodes,
wherein the organic layer comprises the fullerene derivative according to claim 1.

5. An organic photoelectric conversion element comprising a pair of electrodes, at least one of the electrodes being transparent or translucent, and an organic layer disposed between the pair of electrodes,
wherein the organic layer comprises the composition according to claim 2 or 3.
